# EUROPEAN PATENT APPLICATION

(11) **EP 2 647 719 A1**
(43) Date of publication of application: **09.10.2013**
(21) Application number: 13161642.7
(22) Date of filing: 28.03.2013
(51) Int. Cl.: C12P 19/62

(54) **Improved procedure for the production of tiacumicin B**

(30) Priority: 05.04.2012 IT MI20120559
(71) Applicant: OLON S.p.A., 20090 Rodano (MI) (IT)
(72) Inventor: Malcangi, Antonella, 20090 Rodano (MI) (IT); Trione, Guido, 20090 Rodano (MI) (IT)
(74) Representative: Minoja, Fabrizio

(57) **Abstract**

The present invention relates to a process for the production of tiacumicin B which comprises fermentation of a micro-organism of the species *Dactylosporangium aurantiacum* in a culture broth containing polysaccharides such as cellulose and derivatives thereof.

## Description

### Field of invention

The present invention relates to an improved process for the production of tiacumicin B, and particularly to a fermentation method that prevents the degradation of tiacumicin B in the production broth.

### Prior art

Tiacumicin B, also known as fidaxomicin, belongs to a family of macrolactones, produced by Actinomycetes, with a complex history.

Tiacumicin B has the same structure as lipiarmycin, which was isolated and described by Lepetit in 1976 in US 3,978,211 as a novel antibiotic produced by cultivating *Actinoplanes deccanensis* A/10655 ATCC21983.

The first patent claimed the product lipiarmycin and a fermentation method for producing it using *Actinoplanes deccanensis* in a nutrient medium containing assimilable sources of carbon, nitrogen and inorganic salts.

In 1986 Abbott Laboratories filed a novel patent US 4,918,174 relating to the same product, which in this case was called tiacumicin B, obtained from *Dactylosporangium aurantiacum subsp. hamdenensis.*

The producing strain was deposited in the ARS Patent Collection of the Northern Regional Research Center in Peoria, where it was allocated access number NRRL 18085.

The patent claimed the tiacumicins and a process for producing tiacumicins by cultivating *Dactylosporangium aurantiacum subsp. hamdenensis* in a nutrient medium.

More recently, Optimer Pharmaceutical filed a novel patent US 7,507,564 which discloses an improved process for the production of tiacumicins to obtain a yield exceeding 50 mcg/ml.

The process described by Optimer is still based on fermentation of *Dactylosporangium aurantiacum,* but in a fermentation medium containing an adsorbent resin able to adsorb tiacumicin B.

Tiacumicin B is an RNA polymerase inhibitor.

The great interest in tiacumicin B is due to its biological activity against the multidrug-resistant bacterium (hospital superbug) *Clostridium difficile.*

*C. difficile* is a Gram-positive anaerobic spore-forming bacterium which can cause serious intestinal infections by producing toxins.

Tiacumicin B is a narrow-spectrum antibiotic with good activity against Clostridia and minimal activity towards the rest of the intestinal microflora.

Its specificity can be important in reducing the relapse rate observed with broad-spectrum antibiotics, as maintaining the natural balance of the intestinal bacteria helps provide resistance to recolonisation by pathogens.

It was recently demonstrated that tiacumicin B is effective against the multidrug-resistant *Mycobacterium tuberculosis,* and also has good anti-tumoral activity.

### Description of the invention

The present invention relates to an improved process for the production of tiacumicin B, and particularly to a method for preventing the degradation of tiacumicin B which occurs naturally during fermentation, using a stabilising agent that protects tiacumicin B against chemical degradation.

When the fermentation broth is analysed by HPLC, it is observed that the production of tiacumicin B gradually increases during the fermentation time.

At the same time, the appearance of some degradation products is observed during fermentation.

If fermentation is prolonged, the degradation products increase, while the concentration of tiacumicin B declines proportionally.

As frequently observed for other fermentation products, the fermentation environment is unfavourable to chemically unstable compounds.

The variation in pH, the accumulation of catabolites and the presence of enzyme activity are all conditions that contribute to the degradation of unstable compounds.

However, it has surprisingly been observed that the addition of some polysaccharides during fermentation, such as cellulose or a derivative thereof, prevents the degradation of the product.

The present invention relates to a process for the production of tiacumicin B comprising fermentation of a micro-organism of the species *Dactylosporangium aurantiacum* or *Actinoplanes deccanensis* in a fermentation broth containing a cellulose or a derivative thereof.

Microgranular cellulose and microcrystalline cellulose are particularly useful. The cellulose derivatives which can be used include ethyl cellulose, phenyl cellulose and butyl cellulose.

The micro-organism preferably used is *Dactylosporangium aurantiacum subsp. hamdenensis.*

Cellulose is the most abundant biopolymer present in nature. It is a polysaccharide comprising a linear chain consisting of D-glucose units bonded by ß (1→4) linkage in amounts ranging from hundreds to tens of thousands and more.

Cellulose is the structural component of the primary cell wall of green plants, many forms of seaweed and oomycetes.

The product for industrial use is mainly obtained from wood pulp and cotton pulp.

Cellulose is tasteless, odourless, insoluble in water and most organic solvents, chiral and biodegradable. It can be degraded chemically into its glucose units by treatment with concentrated acids at high temperature.

Among the cellulose derivatives, microcrystalline cellulose has numerous applications in the pharmaceutical field, where it is used as excipient, in pellet form, to formulate tablets and capsules.

When the fermentation broth was analysed by HPLC after the addition of cellulose to the medium, it was surprisingly observed that in the presence of this substrate the production of tiacumicin B increases over time, but degradation products do not appear.

The absence, or at least minimal accumulation, of degradation products produces a higher concentration of tiacumicin B in the broth.

Moreover, as the broth mainly contains tiacumicin B as product, the recovery and purification of tiacumicin B from the fermentation broth is more efficient.

The presence of a mainly pure product in the broth obviously simplifies the recovery process and eliminates the need for lengthy, expensive purification stages.

The concentration of cellulose or a derivative thereof in the culture broth ranges between 0.5 g/L and 50 g/L.

The cellulose or derivative thereof preferably has a concentration of 20 g/L in the culture broth.

Cellulose is an inert substrate; the producing micro-organism, *Dactylosporangium aurantiacum,* can grow in the presence of cellulose without metabolising it.

Cellulose is a cheap natural substrate which can easily be added during fermentation at the start of production, and does not interfere with the growth of the micro-organism or production of the antibiotic.

Cellulose or a derivative thereof can be added all at once when the production of tiacumicin B begins, or gradually during fermentation. Cellulose or a derivative thereof can usually be added in a single addition after 72 h fermentation, or added gradually from 72 h onwards.

Cellulose is preferably added as a sterile aqueous suspension.

### Example 1

*Dactylosporangium aurantiacum subsp. hamdenensis* AB718C-41 NRRL18085 was maintained at -180°C (WCB). The stock culture was used to inoculate an Erlenmeyer flask (seed flask) containing 40 ml of vegetative medium VBF-1 (Table 1), which was incubated on a rotary stirrer for 72 h at 30°C and 250 rpm.

**Table 1. vegetative medium VBF-1**

| INGREDIENT | 1 L |
|---|---|
| Yeast extract | 7.5 g |
| Dextrose | 1 g |
| Soluble starch | 24 g |
| Soya peptone | 7.5 g |
| Meat extract | 3 g |
| CaCO₃ | 4 g |
| | |
| pH corrected to 7.3 | |
| Sterilisation 121 °C x 30 min | |

At the end of the incubation, the vegetative culture was transferred aseptically (0.8% of inoculum) to an Erlenmeyer flask containing 30 ml of production medium VPF-1 (table 2), which was incubated on a rotary stirrer at 30°C and 250 rpm.

After 72 h fermentation a sterile suspension of cellulose (Solka Floc BW100) was added aseptically to the fermentation broth to reach a concentration of 20 g/L in the medium. The incubation was then prolonged to 144 h.

| INGREDIENT | 1 L |
|---|---|
| Dextrose | 20 g |
| Soya meal | 10 g |
| Yeast extract | 3 g |
| Soya oil | 1 g |
| K₂HPO₄*7H₂O | 0.05 g |
| MgSO₄*7H₂O | 0.05 g |
| KCl | 0.03 |
| CaCO₃ | 3 g |
| | |
| No pH correction | |
| Sterilisation 121 °C x 30 min | |

### Example 2

*Dactylosporangium aurantiacum subsp. hamdenensis* AB718C-41 NRRL18085 was maintained at -180°C (WCB). The stock culture was used to inoculate a 2L flask containing 450 ml of vegetative medium VBF-1 (Table 1), which was incubated on a rotary stirrer for 72 h at 30°C and 150 rpm.

At the end of the incubation, 1.4% of vegetative inoculum was transferred aseptically to a 20 L fermenter containing 18 L of production medium VPF-2 (Table 3), which was incubated at 30°C, 0.75 vvm, 0.5 bar.

During fermentation, a glucose solution (35%) was added to maintain the glucose concentration in the broth at 3 g/L.

After 72 h fermentation a sterile suspension of cellulose (Solka Floc BW100) (final concentration 20 g/L) was added aseptically to the fermentation broth. The incubation was then prolonged to 230 h.

**Table 3. Production Medium VPF-2**

| INGREDIENT | 1 L |
|---|---|
| Dextrose | 10 g |
| Soya meal | 10 g |
| Casein | 3 g |
| Soya oil | 1 g |
| K₂HPO₄*7H₂O | 0.5 g |
| MgSO₄*7H₂O | 0.5 g |
| KCl | 0.3 |
| CaCO₃ | 3 g |
| | |
| No pH correction | |
| Sterilisation 121 °C x 30 min | |

### Example 3

*Dactylosporangium aurantiacum subsp. hamdenensis* AB718C-41 NRRL18085 was maintained at -180°C (WCB). The stock culture was used to inoculate a flask (capacity 2L) containing 450 ml of vegetative medium VBF-1 (Table 1), which was incubated on a rotary stirrer for 72 h at 30°C and 150 rpm.

At the end of the incubation, 1.4% of vegetative inoculum was transferred aseptically to a 20 L fermenter containing 18 L of production medium VPF-2 (Table 3), which was incubated at 30°C, 0.75 vvm, 0.5 bar.

During fermentation, a glucose solution (35%) was added to maintain the glucose concentration in the broth at 3 g/L.

After 72 h fermentation a sterile suspension of cellulose (Solka Floc BW100) was added aseptically to the fermentation broth every 24 h to reach a final concentration of 20 g/L in the medium after 4 additions. The incubation was then prolonged to 230 h.

## Claims

1. A process for the preparation of tiacumicin B comprising the fermentation of a micro-organism of the species *Dactylosporangium aurantiacum* in a fermentation broth containing cellulose or a derivative thereof.

2. The process according to claim 1 wherein the micro-organism is *Dactylosporangium aurantiacum subsp. Hamdenensis.*

3. The process according to claim 1 or 2 containing cellulose.

4. The process according to claim 1 or 2 wherein the cellulose derivative is microgranular or microcrystalline cellulose.

5. The process according to claim 1 or 2 containing a cellulose derivative selected from the group consisting of ethyl cellulose, butyl cellulose and phenyl cellulose.

6. The process according to claims 1-5 wherein the cellulose or the derivative thereof has a concentration in the fermentation broth ranging from 0.5g/L to 50g/L.

7. The process according to claims 1-5 wherein the cellulose or the derivative thereof has a concentration in the culture broth of 20 g/L.

8. The process according to claims 1-7 wherein the cellulose or the derivative thereof is added at the beginning of the production of tiacumicin B in a single addition or gradually during fermentation.
